# EUROPÄISCHE PATENTSCHRIFT

(11) **EP 2 566 593 B1**
(45) Veröffentlichungstag und Bekanntmachung des Hinweises auf die Patenterteilung: **31.08.2016**
(21) Anmeldenummer: 11707778.4
(22) Anmeldetag: 25.02.2011
(51) Int. Cl.: B01D 3/06, C02F 1/06, C12M 1/107, C02F 11/04

(54) **ABTRENNVERFAHREN UND ABTRENNVORRICHTUNG**
SEPARATION METHOD AND SEPARATION APPARATUS
PROCÉDÉ ET DISPOSITIF DE SÉPARATION

(30) Priorität: 03.05.2010 AT 7512010
(43) Veröffentlichungstag der Anmeldung: 13.03.2013
(73) Patentinhaber: BDI - BioEnergy International AG, 8074 Grambach/Graz (AT)
(72) Erfinder: KIRCHMAYR, Roland, A-6841 Mäder (AT); HARASEK, Michael, A-1160 Wien (AT); MAIER, Christian, A-3281 Oberndorf an der Melk (AT); WALTENBERGER, Reinhold, A-4040 Linz (AT)
(74) Vertreter: Röggla, Harald
(86) Internationale Anmeldenummer: PCT/AT2011/000093
(87) Internationale Veröffentlichungsnummer: WO 2011/137466

(56) Entgegenhaltungen:
- WO-A2-2006/122560
- CN-C- 100 443 137
- DE-A1- 4 341 713
- DE-A1- 10 063 888
- DE-A1- 10 353 728
- DE-A1-102005 047 719
- JP-A- 2003 039 036
- JP-A- 2009 018 211
- US-A- 5 785 852
- US-A1- 2009 050 134

## Beschreibung

Die vorliegende Erfindung betrifft ein Verfahren und eine Vorrichtung zur Abscheidung von Ammoniak bzw. Reduzierung von Ammonium aus zur Biogasproduktion eingesetzten Fermentationsflüssigkeiten oder Gärresten.

Die energetische Verwertung von Abfällen und Nebenprodukten gewinnt in der nachhaltigen Produktion von Konsumgütern immer mehr an Bedeutung. Speziell in der Lebensmittelproduktion können anfallende organische Abfälle durch Anwendung der Biogastechnologie zur (teilweisen) Deckung des Produktionsenergiebedarfs genutzt werden. Somit kann die Verwertung von Abfällen im Rahmen von produktionsintegriertem Umweltschutz und der Erzeugung von erneuerbarer Energie einen Beitrag zur nachhaltigen Entwicklung des Unternehmens leisten. Aus nationaler Sicht sind solche Maßnahmen ebenfalls wünschenswert, da diese einen Beitrag zu den "2020-Klimazielen" der EU liefern.

Das aus den in der Produktion anfallenden Reststoffen gebildete Biogas kann in einem Blockheizkraftwerk (BHKW) in Strom und Wärme umgewandelt werden. Alternativ dazu könnte Biogas aufbereitet werden und direkt fossiles Erdgas substituieren. Durch die Einbindung in einen Betrieb kann eine ganzjährige Abnahme der thermischen Energie sichergestellt werden. So ist der effiziente Einsatz des Energieträgers mit hohen Gesamtwirkungsgraden sichergestellt. Als positiver Nebeneffekt entfällt die aufgrund ihres hohen Wassergehaltes energieintensive Entsorgung dieser Materialien und wertvolle Nährstoffe, wie Stickstoff, Phosphor und Kalium, können mit dem Gärrest auf landwirtschaftliche Flächen rückgeführt werden.

Die weltweit stark steigende Nachfrage nach Fleisch und Fleischprodukten und die damit verbundene Produktion verursachen einen erhöhten Anfall von nicht konsumierbaren tierischen Nebenprodukten. Schlachtabfälle als mögliche neue Substrate industriellen Ursprungs besitzen ein großes Potenzial zur Methanbildung und stehen in keinerlei Konkurrenz zur Lebensmittelproduktion. Diese und viele weitere ähnliche Reststoffe, wie z.B. Abfälle aus der pharmazeutischen Industrie sowie Blutaufbereitung (Plasmaabfälle), Abfälle aus der Eiweiß- und fleischverarbeitenden Industrie und Hefen und Schlempen aus der Ethanolfermentation, können jedoch mit heutigem Stand der Technik nur unter erschwerten Bedingungen in anaeroben Abfallbehandlungsanlagen verarbeitet werden.

In vielen Regionen herrscht durch intensive Landwirtschaft und Tierhaltung bereits eine hohe Stickstoffversorgung der Böden vor. Ein zusätzlicher Nährstoffeintrag kann eine erhöhte Eutrophierung der Gewässer und Belastung des Grundwassers mit sich bringen. Methoden zur Entfernung von Stickstoff aus Biogas-Gärrückständen würden die Aufkonzentrierung des entstehenden Ammoniaks in eine transportfähige Form, die aus der Region verbracht werden kann, ermöglichen und damit eine Entlastung der Umwelt. Damit könnte die Biogasproduktion in Regionen wieder vermehrt angewandt werden, wo sie aufgrund von fehlenden landwirtschaftlichen Flächen nur schwer anwendbar ist.

Ein Hauptproblem auf dem Weg zur flächendeckenden Implementierung der Anaerobtechnik in der energetischen Verwertung von beispielsweise Schlachtabfällen stellt deren hoher Stickstoffgehalt dar. Die Abbauprodukte der im Substrat enthaltenen Stickstoffverbindungen Ammoniak (NH₃) und Ammonium (NH₄⁺) führen ab bestimmten Konzentrationen zu Hemmungen der Mikrobiologie. Diese toxischen Substanzen führen zu einem reduzierten Abbau der eingesetzten Abfälle und in weiterer Folge zu einem instabilen Biogasprozess. Deshalb wurden die stickstoffhältigen Substrate oft an betriebsfremde Biogasanlagen zur Co-Fermentation abgegeben, wodurch die Möglichkeit einer Einbindung der Abwärme des BHKW's in den Produktionsbetrieb entfällt und der Gesamtwirkungsgrad des Schlachtbetriebes sinkt.

Der in dieser Patentschrift beschriebene Prozess stellt eine Schlüsseltechnologie für die anaerobe Verwertung von stickstoffreichen Abfall- und Nebenprodukten dar. Die Entfernung der Stickstofffracht aus dem Biogasprozess gewährleistet einen stabilen und effizienten Prozessverlauf.

Biogas spielt im Kontext von erneuerbarer Energie und betrieblichem Umweltschutz eine bedeutende Rolle. Ein Beispiel dafür stellt die Schlacht- und fleischverarbeitende Industrie dar. Nicht konsumierbare tierische Nebenprodukte bieten in der anaeroben Verwertung ein großes energetisches Potenzial. Mit heutigem Stand der Technik lassen sich solche hoch stickstoffhaltigen Substrate nur unter erschwerten Bedingungen in Biogasanlagen verwerten.

In der anaeroben Fermentation werden die eingesetzten organischen Substanzen (wie beispielsweise Proteine, Nukleinsäuren, Fette und Kohlenhydrate) unter Ausschluss von Sauerstoff durch verschiedene anaerobe Mikroorganismen stufenweise zu kleineren Verbindungen abgebaut. Endprodukte des Biogasprozesses sind Methan (CH₄), Kohlendioxid (CO₂) sowie nicht weiter abbaubare Kohlenstoff- und mineralische Verbindungen. Der in der Biomasse gebundene Stickstoff wird als Ammonium (NH₄⁺) freigesetzt und verbleibt im Gärendprodukt. Zu hohe Konzentrationen können den mikrobiellen Prozess hemmen oder gar toxisch auf die Mikroorganismen wirken. Ammonium steht in einem von Temperatur und pH-Wert abhängigen Gleichgewicht mit Ammoniak (NH₃), das als Zellgift gilt und schon in geringen Konzentrationen inhibierend wirkt.

Damit Rohmaterialien mit hohen Stickstoffkonzentrationen (z.B. proteinreiche Nebenprodukte aus Schlachtbetrieben, Abfälle der Lederindustrie, Reststoffe der Biotreibstoffproduktion usw.) in anaeroben Fermentationsprozessen verarbeitet werden können, ist die Entwicklung neuer Strategien erforderlich.

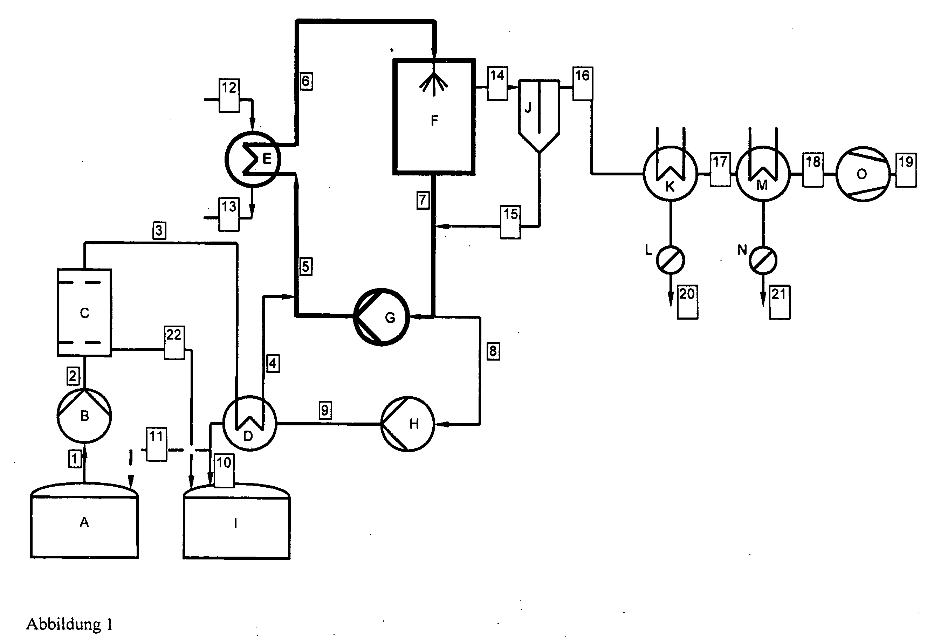

Für die Mikrobiologie im Fermenter ist es wichtig, dass gewisse Mengen an Stickstoffkomponenten zur Verfügung stehen. Diese werden für das Biomassewachstum benötigt; allerdings beträgt die von den Mikroorganismen aufgenommene Menge nur einen Bruchteil der im vorliegenden Substrat enthaltenen Stickstoffmenge. Im Laufe des in Abbildung 1 dargestellten Biogasprozesses werden unter Ausschluss von gelöstem Sauerstoff Polymerverbindungen durch verschiedene heterotrophe anaerobe Mikroorganismen hydrolysiert.

Die stickstoffhaltigen Polymere, Proteine und Nukleinsäuren werden im ersten Schritt zu Aminosäuren, Purinen und Pyrimidinen abgebaut. Im weiteren Verlauf des biologischen Abbauprozesses wird Stickstoff in Form von Ammonium freigesetzt und verbleibt - im Gegensatz zu den biologisch verfügbaren organische Substanzen, die zu Biogas umgewandelt werden - in der Fermentationsflüssigkeit. Dadurch werden die Stickstoffabbauprodukte sogar aufkonzentriert. Mit steigendem pH-Wert und erhöhten Temperaturen im Prozess verschiebt sich das Reaktionsgleichgewicht von Ammonium hin zu Ammoniak.

Ammoniak wirkt sich toxisch auf Bakterien aus, da nahezu alle biologischen Membranen aufgrund der geringen Größe des NH₃-Moleküls sowie dessen Lipidlöslichkeit für Ammoniak durchlässig sind.

Unter den verschiedenen an der anaeroben Verdauung beteiligten Mikroorganismen sind die Methanogenen am wenigsten tolerant gegenüber hohen Konzentrationen an Ammonium und daher am ehesten von Hemmungen und toxischen Wirkungen. Infolge der Hemmung kommt es dann zu geringen Biogasausbeuten und einer Akkumulation von Zwischenabbauprodukten, wie freien flüchtigen Fettsäuren. Neben den verringerten Ausbeuten an erneuerbarer Energie führten die biologischen Prozessstörungen auch zu gesteigerten Geruchsemissionen aus dem Gärendprodukt durch unzureichend abgebaute Substrate.

Zur Lösung des Stickstoffproblems standen bisher prinzipiell mehrere Ansätze zur Diskussion:
- Ausschluss von spezifischen Substraten mit hohem Stickstoffgehalt,
- Verdünnung des stickstoffreichen Substrates oder
- Abtrennung des Stickstoffs aus dem Substrat

Der Ausschluss und die Verdünnung spezifischer Substrate kann zwar für einzelne Anlagenbetreiber in Betracht gezogen werden, als Lösung für die vorliegende Problemstellung der Verwertung von stickstoffreichen Abfällen und Nebenprodukten ist dieser Weg aber nicht zielführend. Eine Verdünnung der Substrate, beispielsweise mit Abwässern mit niedrigen Stickstoffkonzentrationen, führt zu einem enormen Bedarf an zusätzlichem Faulraum. Gleichzeitig würde der Aufwand für die Lagerung und die Transportkosten des Gärrestes vervielfacht. Den vielversprechendsten Ansatz stellt daher die Ausschleusung der Stickstoffkomponenten aus dem Biogasprozess dar.

Diese Vorgangsweise verringert eine Inhibierung der Mikroorganismen durch erhöhte NH₄⁺ / NH₃ Konzentrationen und übt daher folgende positive Einflüsse auf den Biogasprozess aus:
- gesteigerte Methanerträge bei gleichbleibendem Fermentervolumen
- höhere Abbauraten ermöglichen eine höhere Raumbelastung
- stabilerer biologischer Prozess durch Verhinderung der Akkumulation von freien flüchtigen Fettsäuren (FFS), die ebenfalls die Leistungsfähigkeit des Prozesses beeinträchtigen
- Verringerung unerwünschter Geruchsemissionen durch vollständigen Abbau der organischen Masse

Durch eine Abscheidung von signifikanten Mengen des Ammonium-Stickstoffes kann in einer bestehenden Biogasanlage ein verbesserter, stabilerer Betrieb gewährleistet werden und die Abbauleistung und somit der Methanertrag erheblich gesteigert werden. Somit kann die Raumbelastung der Fermenter erhöht werden, wodurch zukünftig das gesamte Substratspektrum sowie dessen maximale Gasausbeute zur Energieerzeugung genutzt werden kann.

Resultierend aus dieser signifikanten Leistungssteigerung ergeben sich entsprechend höhere Ausbeuten an CO₂-neutraler Elektrizität und Wärme aus der gesteigerten Nutzung der BHKW-Kapazitäten. Für die gezielte Entfernung von Ammoniak aus flüssigen Medien wurden vor allem in der Abwassertechnik bereits eine Reihe von Verfahren entwickelt. Einige davon sind teilweise seit geraumer Zeit großtechnisch erprobt und Stand der Technik. Für den relativ jungen Biogassektor wurden diese Technologien adaptiert und können im Bereich der Gärrestaufbereitung eingesetzt werden. Jedoch kommen diese Technologien aufgrund des hohen Energie-, Betriebsmittel- und Substratvorbehandlungsaufwandes lediglich in Ausnahmefällen zur Anwendung.

Generell kann man die Methoden zur Stickstoffentfernung in biologische und physikalisch-chemische Verfahren unterteilen:
Die Nitrifikation/Denitrifikation als biologisches Verfahren stellt eine etablierte Technologie in der biologischen Abwasserreinigung dar. Die Stickstoffentfernung erfolgt hierbei in zwei Teilschritten:

### 1. Nitrifikation

Die Umwandlung von Ammonium zu Nitrat unter *aeroben* Bedingungen wird durch chemolithotrophe nitrifizierende Bakterien durchgeführt. Diese Oxidation erfolgt in zwei Stufen. Bakterien aus der "Nitroso-Gruppe" können Ammoniak oder Ammonium bis zum Nitrit (NO₂⁻) oxidieren (Ammoniumoxidierer). Die weitere Oxidation zum Nitrat (NO₃⁻) erfolgt durch Nitrobacter-Arten oder andere Vertreter der "NitroGruppe« (Nitritoxidierer):

### 2. Denitrifikation

Unter Sauerstoffmangel können verschiedene aerobe Abwasserbakterien organische Verbindungen mit Nitrat an Stelle von O₂ oxidieren. In der Denitrifikation entstehen aus Nitrat gasförmige Endprodukte, hauptsächlich molekularer Stickstoff (N₂). Als Nebenprodukt kann auch Distickstoffoxid (Lachgas, N₂O) freigesetzt werden.

Da die erste der beiden Umwandlungsstufen in aerober Atmosphäre zu erfolgen hat, ist dieses in der Abwassertechnik oftmals erprobte Verfahren für den gegenständlichen Fall der anaeroben Biogasproduktion ungeeignet, da in der aeroben Stufe ein Großteil der chemischen Energie veratmet würde, anstatt zu Biogas umgewandelt zu werden.

Der Anammox-Prozess (Anaerobe Ammonium Oxidation) bietet eine alternative zur klassischen Methode der Nitrifikation/Denitrifikation. Dabei wird Ammonium mit Nitrit unter anaeroben Bedingungen zu molekularem Stickstoff umgesetzt. Trotz einer Vielzahl an Beschreibungen in der Literatur ist dieser Prozess noch nicht als allgemeiner Stand der Technik zu bezeichnen.

Gegenüber den biologischen Verfahren liegt der Vorteil der meisten physikalisch-chemischen Verfahren im Wesentlichen darin, dass vorliegender Ammoniumstickstoff nicht in elementaren Stickstoff umgewandelt wird. Der Stickstoff entweicht daher nicht in die Atmosphäre, sondern steht je nach Verfahren in unterschiedlichen chemischen Verbindungen als Rohstoff bereit.

Die Entfernung der gelösten Stickstoffverbindungen aus dem Fermentationsmedium kann unter Einsatz sogenannter Strippungsverfahren erfolgen. Unter Strippen versteht man allgemein das Austreiben von flüchtigen Verbindungen aus Flüssigkeiten mittels Gas. Durch die Absenkung des Partialdruckes der leichter flüchtigen Komponente tritt diese vom gelösten Zustand in den gasförmigen Zustand in die Gasphase über und reichert sich in der Flüssigkeit ab. Das Grundprinzip stellt sich als eine Einheit mit zwei Hauptverfahrensschritten dar:
- Ammoniakelimination aus dem Substrat mittels Strippgas (Luft bzw. Dampf)
- Regenerierung des Strippgases und Überführung des Ammoniums in einen wieder verwertbaren Stoffstrom

Zur Überführung des gelösten Ammoniaks in die Gasphase (Desorption) haben sich großtechnisch die Luft- und die Dampfstrippung in Füllkörperkolonnen bewährt. Deren Hauptmerkmal besteht darin, dass die zu behandelnde Flüssigkeit mit dem Gasstrom in einen möglichst intensiven Kontakt gebracht wird. Wichtig ist dafür eine möglichst große Austauschfläche. Dies wird durch das Einbringen von Füllkörpern erreicht, an deren Oberfläche sich die Flüssigphase in Form eines Filmes entlang bewegt.

Für den störungsfreien Betrieb einer Strippanlage ist eine weitestgehende Feststoffentfernung Vorraussetzung, da sonst eine Verblockung/Verstopfung der Füllkörperschichtung auftritt. Der Grad der notwendigen Feststoffentfernung hängt jeweils von der Füllkörperart und dem sonstigen Anlagenaufbau ab.

Mit Hilfe von Ionenaustauschverfahren können geladene Inhaltsstoffe (Ionen, z.B. Ammonium NH₄⁺) adsorbtiv gebunden und gegen Ionen mit gleicher Ladung getauscht werden. Dabei kommen vorwiegend synthetische Harze, sogenannte Ionentauscherharze, zur Anwendung. Eine umfangreiche Vorbehandlung des Substrats, insbesondere die Entfernung von Feststoffen, ist notwendige Vorraussetzung. Aufgrund der Struktur des lonentauschermaterials ist dieses relativ empfindlich gegen ein Verstopfen der Hohlräume, beispielsweise durch organische Kolloide, wodurch die Leistung stark reduziert wird. Der Einsatz lohnt sich nach derzeitigem Stand der Technik demnach nur für die Nachbehandlung z.B. nach bereits erfolgter Nanofiltration oder Umkehrosmose zur sicheren Einhaltung von gegebenen Grenzwerten und ist daher für die angestrebte Behandlung des Fermentationsmediums ungeeignet.

Eine in letzter Zeit untersuchte Variante der Ammoniumextraktion ist der Einsatz von extraktiven Membranen in Membrankontaktoren. Unter Membrankontaktoren mit Pervaporation sind neuartige Apparate zur Durchführung von Extraktions-, bzw. Strippprozessen zu verstehen, bei denen der Phasenwechsel an einer Membranoberfläche stattfindet. In herkömmlichen Strippkolonnen befinden sich die flüssige und die gasförmige Phase in direktem Kontakt, wobei die notwendige intensive Durchmischung durch Berieselung von Füllkörpern erzielt wird. In Membrankontaktoren liegen die beiden Phasen durch eine - nur für den gasförmigen Ammoniak durchlässige Membran - voneinander getrennt vor.

Es gibt bei Membranreaktoren jedoch noch eine Reihe von ungeklärten Fragen, so z.B. die nach der Langzeitstabilität und Foulingneigung der Membranen. Insgesamt befinden sich diese Verfahren in einer frühen Entwicklungsstufe und ob eine Umsetzung in die Praxis gelingen wird ist derzeit noch ungewiss.

JP2009018211A offenbart eine Abscheidung von Ammoniak aus Tierexkrementen.

Aufgabe der vorliegenden Erfindung ist es, die Nachteile der obgenannten Verfahren des Standes der Technik zu überwinden und ein Verfahren zur Abscheidung von Ammoniak bzw. Reduzierung von Ammonium aus zur Biogasproduktion eingesetzten Schlachthausabwässern sowie eine Vorrichtung zur Durchführung dieses Verfahrens bereitzustellen. Diese Aufgabe wird erfindungsgemäß durch Anspruch 5 gelöst, in dem bei einem Verfahren der oben angegebenen Art Ammoniak bzw. Ammonium mittels einer. Entspannungsverdampfung reduziert, von den Schlachthausabwässern abgetrennt und als Ammoniak abgeschieden wird. Dabei wird ein Teil des Wassergehalts der im Kreislauf geführten Fermentationsflüssigkeit durch Überhitzung und anschließende Verdüsung in einem unter Vakuum stehenden Behälter verdampft. Dadurch kann ein Teil des Stickstoffes als Ammoniak abgetrennt und anschließend als Wertstoff aufgefangen werden. Der entwickelte Prozess zur Stickstoffentfernung aus zur Biogasproduktion eingesetzten Schlachthausabwässern basiert auf dem Prinzip der Flash-Verdampfung. Bei diesem Verfahren wird die zu behandelnde Flüssigkeit nach einer groben Störstoffabtrennung (z.B. Schneckenpresse, Schwingsieb) und einer Erhitzung über eine Düse in einem unter Unterdruck stehenden Behälter versprüht. Die mit der Druckreduktion verbundene Überschreitung des Siedepunktes führt zu einer spontanen Verdampfung (Flash) an der Oberfläche jedes einzelnen der feinen Tropfen. Diese Verdampfung ist die Triebkraft zur Entfernung von leicht flüchtigen Substanzen, wie z.B. Ammoniak. Der beladene Dampf (Brüden) wird abgeführt und kondensiert. Im Sumpf des Behälters wird die volumsreduzierte Flüssigkeit abgezogen und eventuell nach einer weiteren Erwärmung im Kreis einer weiteren Expansion und damit Verdampfung zugeführt. Diese Technologie wurde ursprünglich für die Meerwasserentsalzung entwickelt, die Behandlung von Medien aus Biogasfermentem stellt demnach ein neues Anwendungsgebiet für derartige Flash-Verdampfer dar.

Vorzugsweise wird bis zu 50%, besonders bevorzugt bis zu 30% des Gesamtstickstoffs der Biogasanlagen-Fermentationsflüssigkeiten oder -Gärreste abgetrennt. Durch diese Entfernung kann ein ungehemmter Biogasprozess sichergestellt werden. Somit können künftig *alle* an z.B einem Schlachtbetrieb oder einem anderen Betrieb mit stickstoffreichen Abfällen anfallenden und für die anaerobe Verwertung geeigneten Ströme bei gleichzeitig verbesserten Abbaugraden energetisch verwertet werden. Dies führt zu einer Steigerung der Produktion erneuerbarer Energien, die bis zu 75% des Energiebedarfs des Industriebetriebes decken kann. Bei Substraten mit sehr hohem Stickstoffgehalt ist es sinnvoll, die Ammoniakentfernung bereits vor die anaerobe Verwertung zu schalten oder in diese zu integrieren, um den Biogasertrag zu maximieren.

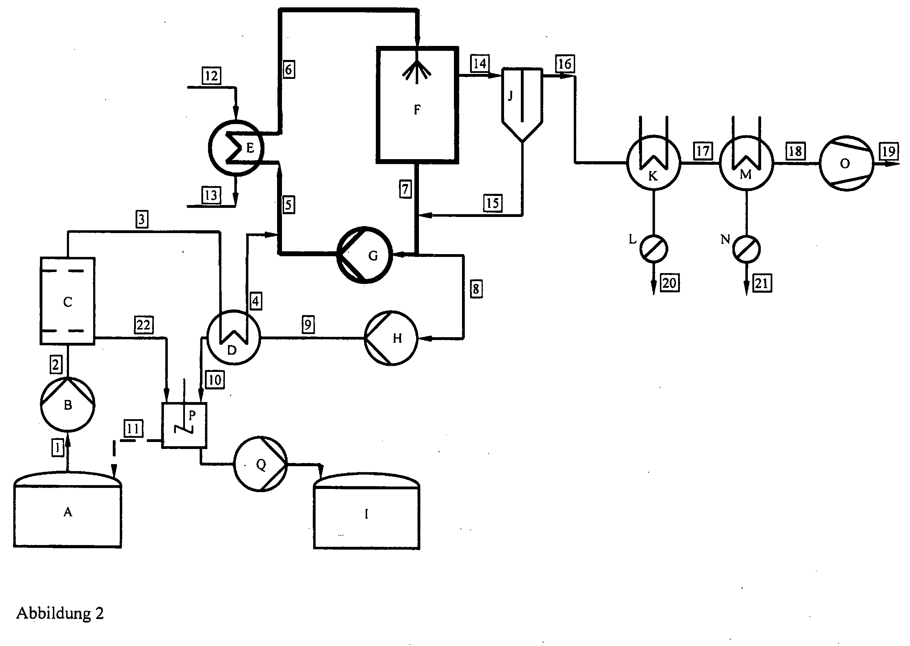

Besonders günstig ist es, wenn die Biogasanlagen-Fermentationsflüssigkeiten oder -Gärreste vor der Entspannungsverdampfung durch die Restwärme von bereits behandeltem Medium vorgewärmt werden. Im Prozess (siehe Abb. 2) wird Material direkt aus einem Biogasfermenter oder dem Gärrestlager entnommen. Das Medium wird in einem Gegenstromwärmetauscher durch 80 °C heißen Ablauf der Anlage auf etwa 70 °C vorgewärmt und in einen internen Rezirkulationskreislauf eingespeist. Die interne Rezirkulation ist eine Stellgröße für die gewünschten Entfemungsraten da diese unter anderem von der Verdampfungsrate abhängig ist welche durch die Rezirkulationsrate eingestellt werden kann. In einem zweiten Wärmetauscher wird das Medium auf die benötigte Eintrittstemperatur des Flash-Verdampfers von etwa 90°C erhitzt. Beim Eintritt in den Flashverdampfer kommt es durch den dort herrschenden Unterdruck zu einer schlagartigen Verdampfung von Wasser, bei dem ebenfalls ein Teil des Ammoniaks in die Gasphase überführt wird. Die mit Ammoniak beladenen Brüdendämpfe aus dem Flash-Verdampfer werden kondensiert und eine wässrige Ammoniaklösung als Produkt aufgefangen. Aus dem Rezirkulationsstrom wird nach dem Flashreaktor kontinuierlich behandeltes Medium abgezogen, im Gegenstromwärmetauscher durch frisches Rohmaterial abgekühlt und in die Biogasanlage rückgeführt.

Die nötige Verdampfungsenergie wird durch einen Gaskessel bereitgestellt. In Folge einer weiteren energetischen Optimierung kann ein Teil dieser Energie durch am Standort anfallende (Ab-)Wärme substituiert werden. Ebenso kann die bei der Kondensation der Brüden frei werdende Abwärme in die Wärmeversorgung der Anlage und des Betriebes eingebunden werden.

Die wässrige Ammoniaklösung kann einerseits als wertvolles Düngemittel in der lokalen Landwirtschaft eingesetzt werden oder andererseits als chemischer Grundstoff zum Beispiel in der Rauchgasreinigung von Kraftwerken zur Entstickung zum Einsatz kommen.

Gemäß der vorliegenden Erfindung betrifft diese eine Vorrichtung zur Abscheidung von Ammoniak bzw. Reduzierung von Ammonium aus Biogasanlagen-Fermentationsflüssigkeiten oder -Gärresten, wobei ein Flash-Verdampfer F mit einem Fermenter A über Leitungen (1, 2, 3, 4, 5, 6) zur Zuführung von Substrat verbunden ist, wobei Brüden aus dem Flash-Verdampfer F über eine Leitung (14) abgezogen und die heiße Flüssigphase über Leitungen (7, 8, 9) entweder über Leitung (11) in den Fermenter A rück- oder über Leitung (10) einem zweiten Fermenter I zugeführt wird.

Günstig ist dabei, wenn in der Leitung (2, 3) aus dem Fermenter A eine Störstoffabtrennung C vorgesehen ist, die leitungsmäßig mit einem Wärmetauscher D für die flüssigen Anteile des Substrats einerseits und über Leitung (22) mit einem zweiten Fermenter I für die festen Anteile des Substrats andererseits verbunden ist.

Bei der erfindungsgemäßen Vorrichtung wird der Wärmetauscher D über Leitung (9) mit heißer Flüssigphase aus dem Flash-Verdampfer F beheizt.

Gemäß der erfinderischen Ausführungsform wird vorgewärmte Flüssigphase aus dem Wärmetauscher D über Leitung (4) in einen Kreislauf, umfassend Leitung (5), Wärmetauscher E, Leitung (6), Flash-Verdampfer F, Leitung (7) und Pumpe G, eingebracht.

Gemäß einer bevorzugten Ausführungsform ist vorgesehen, dass die heiße Flüssigphase über eine Leitung (10) einem Mischtank P zugeführt wird, welcher seinerseits mit den Fermentern A und I leitungsmäßig verbunden ist.

Die vorliegende Erfindung wird nun anhand der beiliegenden Zeichnungen, auf welche sie jedoch nicht beschränkt sein soll, näher erläutert.

In den Fig. 1 und 2 wird jeweils eine mögliche Ausführungsform der vorliegenden Erfindung gezeigt, worin bedeuten:
- A: Fermenter 1
- B: Rohsubstratpumpe
- C: Störstoffabtrennung (Schwingsieb, Schneckenpresse)
- D: Wärmetauscher Vorwärmung
- E: Wärmetauscher
- F: Flash-Verdampfer
- G: Kreislaufpumpe
- H: Abzugspumpe
- I: Fermenter 2
- J: Tropfenabscheidung (z.B. Zyklon)
- K: Kondensator, Stufe 1
- L: Kondensatpumpe 1
- M: Kondensator, Stufe 2
- N: Kondensatpumpe 2
- O: Inertgasfördereinrichtung
- P: Mischtank: Resuspendierung der abgeschiedenen Feststoffe
- Q: Substratpumpe
- 1: Leitung für Rohsubstratstrom
- 2: Leitung für Rohsubstratstrom
- 3: Leitung für Substrat, von festen Störstoffen befreit
- 4: Leitung für vorerwärmtes Substrat
- 5: Leitung für Substrat im Kreislauf des Flash-Verdampfers
- 6: Leitung für Substrat im Kreislauf des Flash-Verdampfers, für Verdampfungsstufe vorgewärmt
- 7: Leitung für Abstrom vom Flash-Verdampfer (Flüssigphase)
- 8: Leitung für Abzug von NH₃-abgereichertem Substrat (Ausschleusung aus dem Kreislaufstrom)
- 9: Leitung für Substratstrom
- 10: Leitung für abgekühltes, an NH₃ abgereichertes Substrat, Einspeisung in Fermenter
- 11: Leitung für abgekühltes, an NH₃ abgereichertes Substrat, optionale Rückeinspeisung in Entnahmefermenter
- 12: Leitung für Heizmedium Zustrom
- 13: Leitung für Heizmedium Abstrom
- 14: Leitung für Brüdenphase aus dem Flash-Verdampfer
- 15: Leitung für aus den Brüden abgeschiedene Tropfen
- 16: Leitung für Brüdenstrom
- 17: Leitung für restliche Brüden, nach erster Kondensatorstufe
- 18: Leitung für Inertgase, nach zweiter Kondensatorstufe
- 19: Leitung für Inertgase zu Gasbehandlung (z.B. Biofilter)
- 20: Leitung für Kondensatstrom der ersten Kondensatorstufe
- 21: Leitung für Kondensatstrom der zweiten Kondensatorstufe, NH₃-angereichert
- 22: Leitung für aus dem Rohsubstrat abgetrennten Feststoffanteil

Gemäß Fig. 1 wird das zu behandelnde Rohsubstrat mittels der Pumpe **B** direkt dem Biogasfermenter oder Gärrestlager **A** entnommen. Im Medium enthaltene feste Stoffe werden in der Störstoffabtrennung **C** (z.B. Schneckenpresse, Schwingsieb) aus der Flüssigkeit abgeschieden und in den Fermenter **I** eingetragen. Der flüssige Anteil des Substrats (Leitung **3**) wird im Gegenstrom mit fertig behandeltem Substrat aus Leitung **9** im Wärmetauscher **D** (z.B. Spiralwärmetauscher) auf eine Temperatur von ca. 65 bis 75°C vorerwärmt und dann über Leitung **5** in den internen Kreislaufstrom eingespeist. In einem zweiten Wärmetauscher **E** (z.B. Kondensator, Frischdampf aus Leitung **12**) wird das Medium auf die für den Verdampfungsprozess benötigte Eintrittstemperatur von ca. 80 bis 95°C erhitzt.

Beim Versprühen des Mediums in den Flash-Verdampfer wird der Vordruck in Leitung 6 von ca. 1,5 bis 3,0 bara über den Druckverlust der Sprühdüse auf den Betriebsdruck im Flash-Verdampfer von ca. 500 bis 700 mbar entspannt. Beim Eintritt in den Flash-Verdampfer kommt es aufgrund des dort herrschenden Unterdrucks zu einer schlagartigen Unterschreitung des Siedepunkts und somit zu einer spontanen Verdampfung an der Oberfläche der Tropfen des Sprühnebels. Diese Verdampfung ist die Triebkraft zur Entfernung von leicht flüchtigen Substanzen, wie z.B. Ammoniak und gelöstem Kohlendioxid. Auch ein Teil des Wassers im Substrat wird in diesem Schritt verdampft. Die mit NH₃ und CO₂ beladenen Brüden werden über Leitung **14** einer Tropfenabscheidung **J** (z.B. Zyklon) zugeführt. Aus dem Gasstrom abgeschiedene Flüssigkeit wird über Leitung **15** der aus dem Sumpf der Verdampferstufe abgezogenen Flüssigphase in Leitung **7** wieder zugeführt. Der Kreislaufstrom der Leitungen **5**, **6**, **7** wird über die Pumpe **G** aufrechterhalten. Die Umlaufzahl als Verhältnis von Kreislaufstrom zu Frischsubstrat kann über die Pumpenleistung von **G** eingestellt werden und dient so als Regelgröße für die NH₃-Entfernungsrate.

Behandeltes Substrat wird kontinuierlich aus dem Kreislaufstrom abgezogen (Leitung **8**). Dieses an NH₃ und CO₂ abgereicherte Substrat fällt bei einer Temperatur von ca. 75 bis 90°C an. Vor der Verpumpung (Pumpe **H**) in den Fermenter **I** wird der Wärmeinhalt des Stroms von Leitung **9** mittels Gegenstromwärmetauscher **D** (z.B. Spiralwärmetauscher) auf ca. 40 bis 55°C abgekühlt und im Gegenzug Rohsubstrat aufgewärmt. Die einzusetzende Heizleistung kann durch diese Energieintegration massiv reduziert werden; weiters kann das Medium in den Fermenter eingeleitet werden, ohne die gasbildende Mikrobiologie durch Übertemperaturen zu gefährden. Das abgekühlte, behandelte Substrat wird dann entweder über Leitung **10** in den Fermenter **I** oder, falls gewünscht alternativ oder zusätzlich, über Leitung **11** in den Fermenter oder das Gärrestlager A verbracht.

Die mit NH₃ beladenen Brüden aus dem Flash-Verdampfer **14** werden einer Brüdenkondensation zugeführt. Nach Abkühlung auf ca. 50 bis 60°Cim Kondensator **K** kann eine wässrige Ammoniaklösung **21** von 20 bis 80 g/kg NH₃ der weiteren stofflichen Verwertung (z.B. Düngemittelherstellung, Einsatz als SNCR-Reagens in der Kraftwerkstechnik, usw.) zugeführt werden.

In der gezeigten Ausführungsform ist die Brüdenkondensation zweistufig ausgeführt. In der ersten Kondensationsstufe **K** werden die Brüden auf ca. 75 bis 85°C abgekühlt. Das erste Kondensat fällt in mit einer niedrigen Konzentration von ca. 5 bis 10 g/kg NH₃ an (Strom **20**). Nach weiterer Abkühlung auf ca. 50 bis 60°C im Kondensator **M** kann eine wässrige Ammoniaklösung **21** mit höherer Ammoniakkonzentration von 40 bis 80 g/kg NH₃ der weiteren stofflichen Verwertung (z.B. Düngemittelherstellung, Einsatz als SNCR-Reagens in der Kraftwerkstechnik, usw.) zugeführt werden. Alternativ (nicht gezeigt) ist auch eine einstufige Brüdenkondensation möglich.

Die bei den für die Kondensationsstufen angegebenen Temperaturen nicht kondensierbaren Gase werden via Leitung **18** über eine Inertgasfördereinrichtung **O** abgesaugt, welche auch den Systemdruck von 500 bis 700 mbar im Flash-Verdampfer aufrechterhält.

In einer besonderen Ausführungsform ist diese Inertgasfördereinrichtung **O** zur Absaugung der nicht kondensierbaren Gase als Wasserstrahlpumpe mit Wasser als Umlaufmedium ausgeführt. Dies hat den Vorteil, dass die abgesaugten, nicht kondensierbaren Gase mit dem Wasserstrahlpumpenwasser gleichzeitig gewaschen werden und damit von restlichen Spuren an NH₃ befreit werden.

Gemäß Fig. 2 wird das abgekühlte, behandelte Substrat in einen Mischtank **P** verbracht, wo es mit den festen Stoffen aus der Störstoffabtrennung **C** (z.B. Schneckenpresse, Schwingsieb) vermischt wird, wonach das Medium entweder über Leitung **11** in den Fermenter **A** oder, falls gewünscht alternativ oder zusätzlich, über Leitung **10** und mittels Substratpumpe **Q** in den Fermenter **I** verbracht wird.

## Patentansprüche

1. Vorrichtung zur kontinuierlichen Abscheidung von Ammoniak bzw. Reduzierung von Ammonium aus Biogasanlagen-Fermentationsflüssigkeiten oder -Gärresten, umfassend einen Flash-Verdampfer F, der über Leitungen, umfassend Rohsubstratleitungen (1, 2) mit einer zwischengeschalteten Rohsubstratpumpe B, eine anschließende Störstoffabtrennung C sowie eine daran anschließende Leitung (3) für von festen Störstoffen befreites Substrat, einen Wärmetauscher D, eine an den Wärmetauscher D anschließende Leitung (4) für vorerwärmtes Substrat, mit einem Fermenter oder Gärrestlager A zur Zuführung von Substrat verbunden ist, wobei das vorerwärmte Substrat in einen Kreislauf eingebracht wird, der eine Kreislauf-Leitung (5) für Substrat, einen Wärmetauscher E zur Erhitzung des Substrats auf eine Eintrittstemperatur in den Flash-Verdampfer F sowie eine Kreislauf-Leitung (6) für erhitztes Substrat, den Flash-Verdampfer F, eine Leitung (7) vom Flash-Verdampfer F und eine daran anschließende Pumpe G umfasst, wobei Brüden aus dem Flash-Verdampfer F über eine Brüdenphase-Leitung (14) abziehbar ist und die heiße Flüssigphase über Leitungen, umfassend die Leitung (7) vom Flash-Verdampfer zur Pumpe G im Kreislaufstrom, eine Abzug-Leitung (8), eine Abzugspumpe H sowie eine Substratstrom-Leitung (9), entweder über eine Rückeinspeise-Leitung (11) in den Fermenter oder das Gärrestlager A rückführbar oder über eine Einspeise-Leitung (10) einem zweiten Fermenter oder Gärrestlager I zuführbar ist, **dadurch gekennzeichnet dass** der Wärmetauscher D über die Substratstrom-Leitung (9) mit heißer Flüssigphase aus dem Flash-Verdampfer F beheizt wird, und der Flash-Verdampfer F über die Brüdenphase-Leitung (14), eine Tropfenabscheidung J und Kondensatoren K und M mit einer Inertgasfördereinrichtung O verbunden ist.

2. Vorrichtung nach Anspruch 1, **dadurch gekennzeichnet, dass** die Inertgasfördereinrichtung O als Wasserstrahlpumpe ausgeführt ist.

3. Vorrichtung nach Anspruch 1 oder 2, **dadurch gekennzeichnet, dass** die in der Leitung (2, 3) aus dem Fermenter A vorgesehene Störstoffabtrennung C leitungsmäßig mit dem Wärmetauscher D für die flüssigen Anteile des Substrats einerseits und über Leitung (22) mit dem zweiten Fermenter I für die festen Anteile des Substrats andererseits verbunden ist.

4. Vorrichtung nach einem der Anspruche 1 bis 3, **dadurch gekennzeichnet, dass** die heiße Flüssigphase über die Leitung (10) einem Mischtank P zugeführt wird, welcher seinerseits mit den Fermentern A und I leitungsmäßig verbunden ist.

5. Verfahren in einer Vorrichtung nach Anspruch 1 zur kontinuierlichen Abscheidung von Ammoniak bzw. Reduzierung von Ammonium aus Biogasanlagen-Fermentationsflüssigkeiten oder -Gärresten, insbesondere jenen, die aus der Vergärung von Schlachtabfällen erzeugt werden, wobei Ammoniak bzw. Ammonium mittels einer Entspannungsverdampfung reduziert, von den Biogasanlagen-Fermentationsflüssigkeiten oder -Gärresten abgetrennt und als Ammoniak abgeschieden wird, **dadurch gekennzeichnet, dass** die Biogasanlagen-Fermentationsflüssigkeiten oder -Gärreste vor der Entspannungsverdampfung durch die Restwärme von behandeltem Medium vorgewärmt werden, danach vor der Entspannungsverdampfung auf eine Temperatur von 80°C bis 95°C erhitzt und von einem Vordruck vor der Entspannungsverdampfung von 1,5 bar bis 3,0 bar durch Versprühen auf einen Betriebsdruck von 500 mbar bis 700 mbar entspannt werden und das behandelte Medium nach Wärmetausch mit den Biogasanlagen-Fermentationsflüssigkeiten oder -Gärresten einem Fermenter oder Gärrestlager zugeführt wird.

## Claims

1. A device for continuously separating ammonia or reducing ammonium, respectively, from biogas plant fermentation liquids or biogas plant fermentation residues, comprising a flash evaporator F which is connected with a fermenter or a fermentation residue storage A for supplying a substrate via lines comprising lines for raw substrate flow (1, 2) with an interposed raw substrate pump B, a subsequent impurity separation C and an adjoining line (3) for substrate freed of solid impurities, a heat exchanger D, and a line (4) for pre-heated substrate attached to the heat exchanger D, wherein the pre-heated substrate is introduced into a circuit comprising a circuit-line (5) for substrate, a heat exchanger E for heating the substrate to an entry temperature of the flash evaporator F, and a circuit-line (6) for heated substrate, the flash evaporator F, a line (7) originating from the flash evaporator F and a pump G joined thereto, wherein exhaust vapour can be withdrawn from the flash evaporator F by way of a line (14) for an exhaust vapour phase and wherein the hot liquid phase can either be returned through a feed-back line (11) into the fermenter or the fermentation residue storage A via lines comprising line (7) leading from the flash evaporator to the pump G in the circuit flow, a line (8) for withdrawal, a withdrawal pump H and a line (9) for substrate flow, or can be supplied via a feed line (10) into a second fermenter or fermentation residue storage I, **characterized in that** the heat exchanger D is heated via line (9) for substrate flow with a hot liquid phase from the flash evaporator F, and the flash evaporator F is connected with an inert gas feed device O via the line (14) for an exhaust vapour phase, a droplet separation device J as well as condensators K and M.

2. The device according to claim 1, charcterized in that the inert gas feed device O is a water jet pump.

3. The device according to claim 1 or 2, **characterized in that** the impurity separation C provided in the line (2, 3) from the fermenter A is flow-connected with the heat exchanger D for the liquid portions of the substrate, on the one side, and via line (22) with the second fermenter I for the solid portions of the substrate, on the other side.

4. The device according to any of claims 1 to 3, **characterized in that** the hot liquid phase is supplied via line (10) to a mixing tank P, which in turn is flow-connected with the fermenters A and I.

5. A method in a device according to claim 1 for continuously removing ammonia or reducing ammonium, respectively, from biogas plant fermentation liquids or fermentation residues, in particular those, which are produced in the fermentation of offal, wherein ammonia or ammonium, respectively, is reduced by way of flash evaporation, is separated from the biogas plant fermentation liquids or biogas plant fermentation residues, and is removed as ammonia, **characterized in that** the biogas plant fermentation liquids or biogas plant fermentation residues are pre-heated before flash evaporation by residual heat of already treated medium, are then heated before flash evaporation to a temperature of from 80°C to 95°C, and are depressurized by spraying from a pre-pressure before flash evaporation of about 1.5 bar to 3.0 bar to an operational pressure of from 500 mbar to 700 mbar, and that the medium thus treated is supplied to a fermenter or a fermentation residue storage after heat exchange with the biogas plant fermentation liquids or fermentation residues.

## Revendications

1. Dispositif pour l'élimination continue de l'ammoniac ou pour la réduction de l'ammonium issu de liquides ou de résidus de fermentation d'installations de biogaz, comprenant un évaporateur flash F, qui est relié, par l'intermédiaire de conduites, comportant des conduites de substrat brut (1, 2) pourvues d'une pompe à substrat brut B intercalée, un dispositif de séparation d'impuretés C adjacent ainsi qu'une conduite (3) raccordée à ce dernier pour un substrat exempt d'impuretés solides, un échangeur de chaleur D, une conduite (4) raccordée à l'échangeur de chaleur D pour le substrat préchauffé, à un fermenteur ou à une cuve de fermentation A pour amener le substrat, le substrat préchauffé étant introduit dans un circuit, qui comporte une conduite (5) de circuit pour le substrat, un échangeur de chaleur E destiné à chauffer le substrat à une température d'entrée dans l'évaporateur flash F ainsi qu'une conduite de circuit (6) pour le substrat chauffé, l'évaporateur flash F, une conduite (7) partant de l'évaporateur flash F et une pompe G raccordée à ce dernier, la vapeur provenant de l'évaporateur flash F pouvant être évacuée par l'intermédiaire d'une conduite de phase vapeur (14) et la phase liquide chaude pouvant soit être ramenée dans le fermenteur ou la cuve de fermentation A en passant par une conduite de ré-injection (11) soit être amenée à un deuxième fermenteur ou à une deuxième cuve de fermentation I en passant par une conduite d'injection (10) par l'intermédiaire de conduites, comprenant la conduite (7) allant de l'évaporateur flash à la pompe G dans le courant de recyclage, une conduite d'évacuation (8), une pompe d'évacuation H ainsi qu'une conduite de courant de substrat (9), **caractérisé en ce que** l'échangeur de chaleur D est chauffé par l'intermédiaire de la conduite de courant de substrat (9) au moyen de la phase liquide chaude provenant de l'évaporateur flash F, et l'évaporateur flash F est relié à un dispositif de refoulement de gaz inerte 0 par l'intermédiaire de la conduite de vapeur (14), d'un dispositif de séparation par gouttes J et de condensateurs K et M.

2. Dispositif selon la revendication 1, **caractérisé en ce que** le dispositif de refoulement de gaz inerte 0 est réalisé sous la forme d'une pompe à jet d'eau.

3. Dispositif selon la revendication 1 ou 2, **caractérisé en ce que** le dispositif de séparation d'impuretés C prévu dans la conduite (2, 3) partant du fermenteur A est relié d'une part par des conduites à l'échangeur de chaleur D pour les fractions liquides du substrat et d'autre part au deuxième fermenteur I pour les fractions solides du substrat par l'intermédiaire de la conduite (22).

4. Dispositif selon l'une quelconque des revendications 1 à 3, **caractérisé en ce que** la phase liquide chaude est amenée par la conduite (10) à un réservoir de mélange P, lequel est relié pour sa part par des conduites aux fermenteurs A et I.

5. Procédé dans un dispositif selon la revendication 1 pour l'élimination continue de l'ammoniac ou la réduction de l'ammonium issu de liquides ou de résidus de fermentation d'installations de biogaz, en particulier ceux qui sont produits à partir de la digestion de déchets d'abattoir, l'ammoniac ou l'ammonium étant réduit au moyen d'une évaporation flash, séparé des liquides ou des résidus de fermentation d'installations de biogaz et éliminé sous forme d'ammoniac, **caractérisé en ce que** les liquides ou résidus de fermentation d'installations de biogaz sont préchauffés avant l'évaporation flash par la chaleur résiduelle du milieu traité, puis chauffés avant l'évaporation flash à une température de 80 °C à 95 °C et détendus par une pré-pression avant l'évaporation flash de 1,5 bar à 3,0 bar par atomisation à une pression de service de 500 mbar à 700 mbar et le milieu traité est amené à un fermenteur ou à une cuve de fermentation après l'échange de chaleur avec les liquides ou les résidus de fermentation d'installations de biogaz.
